(19) Europäisches Patentamt

European Patent Office

Office européen des brevets

(11) **EP 1 561 479 A1**

(12) **EUROPEAN PATENT APPLICATION**

(43) Date of publication:
**10.08.2005 Bulletin 2005/32**

(51) Int Cl.$^7$: **A61L 15/26**

(21) Application number: **05002021.3**

(22) Date of filing: **01.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **03.02.2004 JP 2004026761**
**03.02.2004 JP 2004026763**
**03.02.2004 JP 2004026773**

(71) Applicant: **Nitto Denko Corporation**
**Ibaraki-shi, Osaka 567-8680 (JP)**

(72) Inventors:
• **Suzuki, Seishi Nitto Denko Corporation**
**Ibaraki Osaka 567-8680 (JP)**

• **Okada, Katsuhiro Nitto Denko Corporation**
**Ibaraki Osaka 567-8680 (JP)**
• **Sasaki, Yasuyuki Nitto Denko Corporation**
**Ibaraki Osaka 567-8680 (JP)**
• **Yoshikawa, Toshiyuki Nitto Denko Corporation**
**Ibaraki Osaka 567-8680 (JP)**

(74) Representative: **Albrecht, Thomas, Dr. et al**
**Kraus & Weisert**
**Patent- und Rechtsanwälte**
**Thomas-Wimmer-Ring 15**
**80539 München (DE)**

(54) **Urethane film base material for adhesive skin patch**

(57) To have acceptable moisture permeability and enable prevention of deformation due to swelling, the filmbasematerial for an adhesive skin patch includes an ether-based urethane resin obtained from at least one member selected from the group consisting of polyoxytetramethylene glycol, butanediol, polyethylene glycol, and polypropylene glycol as a diol component, and methylene diphenyl-diisocyanate as an isocyanate component. The film base material for an adhesive skin patch has a moisture permeability of preferably 800 to 4,000 g/m$^2$ · 24hrs. The adhesive skin patch can be produced by forming a pressure-sensitive adhesive layer on one side of the film base material for an adhesive skin patch.

**Description**

BACKGROUND OF THE INVENTION

Field of the Invention

The present invention relates to a film base material for an adhesive skin patch and an adhesive skin patch. More particularly, the present invention relates to a film base material for an adhesive skin patch and an adhesive skin patch having moisture permeability.

Description of a Related Art

Pressure-sensitive adhesive tapes for medical use and for hygienical materials must be able to prevent invasion of water, bacteria, and viruses and so on from outside and have sufficient flexibility to follow up the curve or motion of skin. For this reason, elastomer thin films having low elastic moduli similar to that of skin have been generally used as supports for such pressure-sensitive adhesive tapes. Also, pressure-sensitive adhesive tapes for medical use and for hygine materials, for example, dressings are required to have excellent moisture permeability so that moisture due to perspiration through the skin can be transpired to the outside. If the pressure-sensitive adhesive tapes have poor moisture permeability, the moisture generated by the skin is pooled between the skin and a pressure-sensitive adhesive layer of the tape. This results in a decrease in adhesion of the pressure-sensitive adhesive layer, so that the fixing function of the pressure-sensitive adhesive layer is lost. Also, the moisture pooled on the surface of skin causes maceration of the skin, so that skin disorders tend to occur.

To impart films with moisture permeability, the films are formed from materials that are mixed with inorganic fine powder and the like and elongated to form a number of fine pores therein or films having a number of fine pores are formed by foaming upon film forming. Also, films having a number of fine pores are formed by forming through holes after the films are formed.

Incidentally, when films that have such fine pores are applied to the skin, clogging of the fine pores occurs due to sweat, dirt, dust and so on, resulting in a decrease in moisture permeability. As a result, studies on films that have no pores, i.e., pore-less films having moisture permeability come to be made. For example, pore-less, moisture permeable polyurethane films have been proposed.

However, conventional moisture permeable polyurethane films may in some cases suffer a considerable decrease in strength due to absorption of a large amount of moisture or swell as a result of excessive moisture absorption.

SUMMARY OF THE INVENTION

Under the circumstances, the present invention has been made and it is an object of the present invention to provide a film base material for an adhesive skin patch made of an ether-based urethane resin having excellent moisture permeability and to provide an adhesive skin patch made from the film base material for an adhesive skin patch.

To attain the above-mentioned object, the film base material for an adhesive skin patch of the present invention comprises an ether-based urethane resin obtained from at least one member selected from the group consisting of polyoxytetramethylene glycol, butanediol, polyethylene glycol, and polypropylene glycol as a diol component, and methylene diphenyl-diisocyanate as an isocyanate component.

Here, the diol components that can be used include polyoxytetramethylene glycol, and polyethylene glycol and/or polypropylene glycol.

For example, the above-mentioned ether-based urethane resin may contain 5 to 60% by weight of the polyoxytetramethylene glycol and 10 to 50% by weight of the polyethylene glycol.

Preferably, the film base material for an adhesive skin patch of the present invention is a film base material consisting of a urethane based resin and has a water swelling ratio of 5% or less expressed as a change in length when immersed in water at 40°C for 5 minutes.

Here, it is more preferable that the above-mentioned water swelling ratio is substantially 0%.

The film base material for an adhesive skin patch of the present invention may have a thickness of 10 $\mu$m to 50 $\mu$m.

Further, the film base material for an adhesive skin patch of the present invention may comprise an ether-based urethane resin film having a thickness of 10 $\mu$m to 50 $\mu$m, and have a tensile strength in at least one direction of 5 to 30N/20mm-width, an elongation in at least one direction of 400% to 1,000%, and a tear strength in at least one direction of 400 to 1,000 N/cm-thickness.

Still further, the film base material for an adhesive skin patch of the present invention may have a 100% modulus in at least one direction of 1 to 5 N/20 mm-width.

In the present invention, it is preferable that the film base material for an adhesive skin patch of the present invention has a moisture permeability of 800 to 4,000 $g/m^2 \cdot 24hrs$.

The adhesive skin patch of the present invention is characterized by having a pressure-sensitive adhesive layer on one side of any one of the above-mentioned film base materials for an adhesive skin patch.

Here, the above-mentioned pressure-sensitive adhesive layer preferably comprises at least one pressure-sensitive adhesive selected from the group consisting of an acrylic pressure-sensitive adhesive consisting mainly of a (meth)acrylic acid ester, a silicone pressure-sensitive adhesive consisting mainly of polyorganosiloxane, and a urethane pressure-sensitive adhesive consisting mainly of polyether polyurethane and/or polyester polyurethane.

DETAILED DESCRIPTION

The film base material for an adhesive skin patch of the present invention is a film base material made of a urethane resin, for example, a film base material made of an ether-based urethane resin obtained from a diol component and an isocyanate component. Here, at least one kind selected from the group consisting of polyoxytetramethylene glycol (hereinafter, sometimes abbreviated as "OTMG"), butanediol (hereinafter, sometimes abbreviated as "BD"), polyethylene glycol (hereinafter, sometimes abbreviated as "PEG"), and polypropylene glycol (hereinafter, sometimes abbreviated as "PPG") is used as the diol component and methylene diphenyl-diisocyanate (diphenylmethane-diisocyanate) (hereinafter, abbreviated as "MDI") is used as the isocyanate component.

Note that the term or concept "film" as used herein includes sheet and the term or concept "sheet" as used herein includes film.

It is preferable that polyoxytetramethylene glycol, polyethylene glycol, and polypropylene glycol used as the diol component be selected so that they have appropriate molecular weights depending on applications. Preferable examples thereof include those having a weight-average molecular weight in the range of, for example, 500 to 3,000.

In the present invention, it is preferable that polyoxytetramethylene glycol, and polyethylene glycol and/or polypropylene glycol be used together. In particular, it is preferable that the ether-based urethane resin contains 5 to 60% by weight of the polyoxytetramethylene glycol and 10 to 50% by weight of the polyethylene glycol. More preferably, the ether-based urethane resin contains 5 to 45% by weight of the polyoxytetramethylene glycol and 20 to 45% by weight of the polyethylene glycol.

Further, use of a random copolymer of, for example, polyoxytetramethylene glycol and polyethylene glycol as the diol component ensures high moisture permeability while preventing water swellability, so that when water swellability is to be taken into consideration, it is desirable that a random copolymer of OTMG and PEG is used.

In the present invention, a chain extender may also be used. Conventional chain extenders may be used. Examples thereof include diols such as ethylene glycol, propylene glycol, and butanediol, and diamines such as ethylenediamine and triethylenediamine.

In the present invention, additives that are usually used in films, for example, ultraviolet absorbents, antioxidants, fillers, pigments, colorants, flame retardants, antistatic agents and so on may be added as necessary. The additives may be used in amounts usually used depending on their kind.

The ether-based urethane resin can be polymerized by using, for example, a one-shot method or a prepolymer method. Further, in the case of bulk polymerization without using solvents, the polymerization may be performed in solvents in order to decrease viscosity.

Hereinafter, bulk polymerization will be described in detail. That is, a diol component is charged in a reactor, the temperature is adjusted to 50 to 80°C, and an isocyanate component is added while stirring to cause urethanation to occur. Further, a chain extender is added and reacted and then the reaction product is transferred to a tray, retained at 100 to 150°C for 4 hours or more to complete the reaction, thereby obtaining bulky ether-based urethane resin.

Then, the bulky ether-based urethane resin is pulverized and formed into pellets. The resin pellets are molten and then molded into a sheet by using a T-die extruder or an inflation die extruder to form a film base material made of the ether-based urethane resin. Note that the film base material extruded into a sheet usually is rolled up. Alternatively, by using calendaring the ether-based urethane resin is rolled, elongated and made into a sheet between two hot rolls to form a film base material made of the ether-based urethane resin. The film base material is rolled up as necessary. Also, the film base material made of the ether-based urethane resin may be formed by dissolving the resin pellets in a solvent such as N,N-dimethylamide and coating the solution on a release liner such as, for example, polyester film by using a bar coater or the like, and drying the resultant to remove the solvent.

In the present invention, the thickness of the film base material made of the ether-based urethane resin is preferably in the range of 10 μm to 150 μm in the case of a pressure-sensitive adhesive sheet (adhesive skin patch) for medical use or for hygienical materials. If the thickness of the film is less than 10 μm, the pressure-sensitive adhesive sheet tends to be difficult to handle when it is applied to the skin or peeled from the skin; the handleability of the pressure-sensitive adhesive sheet is decreased to levels at which its use is practically impossible in ordinary methods in which it is used. On the other hand, if the thickness of the film is greater than 150 μm, sufficient moisture permeability

is not obtained, so that the pressure-sensitive adhesive sheet is unsuitable for an adhesive skin patch so far as it is to be applied to the skin. When the adhesive skin patch is used for dressing applications, it is particularly preferable that the thickness of the film is in the range of 20 μm to 60 μm. Further, in applications where a thin adhesive skin patch is necessary, the thickness of the film is preferably 10 μm to 50 μm, more preferably 25 μm to 35 μm.

Note that the film base material for an adhesive skin patch may be of a multi-layer construction, for example, a laminate of ether-urethane resin film.

It is preferable that the film base material for an adhesive skin patch of the present invention has a water swelling ratio of 5% or less expressed as a change in length when immersed in water at 40°C for 5 minutes. More preferably, the water swelling ratio of the film base material is substantially 0%. Here, "substantially 0%" means that an amount of water to such an extent that moisture absorbed from the atmosphere when the film base material is stored in ordinary state or at the time of production may be ignored, and that in consideration of measurement errors, the measurement range measured by the test method described hereinbelow is on the same order as 0%.

For example, an ether-based urethane resin obtained by using at least one kind selected from the group consisting of polyoxytetrametylene glycol, butanediol, polyethylene glycol, and polypropylene glycol as a diol component, and methylene diphenyl-diisocyanate as an isocyanate component can form films having a water swelling ratio of 5% or less by appropriately selecting kinds of blends, blending amounts and so on. Alternatively, in the case where the material does not have to be limited to the ether-based urethane resin, ester-based urethane resins in which a dicarboxylic acid component such as adipic acid is blended may also be used since such urethane resins can also form films having a water swelling ratio of 5% or less.

The film base material for an adhesive skin patch of the present invention has moisture permeability (Dry method) of a 30-μm-thick film of preferably 800 g/$m^2$·24 hrs or more and 4,000 g/$m^2$·24 hrs or less, more preferably 1,000 g/$m^2$·24 hrs or more and 4,000 g/$m^2$·24 hrs or less, and particularly preferably 1,300 g/$m^2$·24 hrs or more and 4,000 g/$m^2$·24 hrs or less.

Further, it is preferable that the film base material for an adhesive skin patch has high moisture permeability in a swelled state. For example, it is preferable that the film base material has moisture permeability as measured by, for example, a filter paper cover method (Wet method) of 3,000g/$m^2$·24 hrs or more.

Here, the term "moisture permeability" of film or the like means an amount of water vapor that passes through 1 $m^2$ of a filmor the like under predetermined conditions. Specifically, a method for measuring the moisture permeability of a film is shown in the examples described hereinbelow. For example, the moisture permeability (Dry method) is obtained by charging a predetermined amount of water in a vessel having a predetermined size of aperture, sealing the opening of the vessel with a film, allowing the vessel to stand under the conditions of a temperature of 40°C and a relative humidity of 30%RH for 24 hours, and measuring an amount of water decreased per unit $m^2$. The higher the moisture permeability of a film, the film causes less non-breathing.

The film base material for an adhesive skin patch of the present invention, in a thickness of 10 μm to 50 μm, has a tensile strength in at least one direction of preferably 5 to 30 N/20 mm-width, an elongation in at least one direction of preferably 400% to 1,000%, and a tear strength in at least one direction of preferably 400 to 1, 000 N/cm-thickness. This design allows the film base material for an adhesive skin patch to achieve flexibility and followability to the skin. Further, the film base material for an adhesive skin patch has a tensile strength in at least one direction of more preferably 10 to 22 N/20 mm-width, an elongation in at least one direction of more preferably 600% to 900%, and a tear strength in at least one direction of more preferably 550 to 850 N/cm-thickness.

The film base material for an adhesive skin patch of the present invention, in a thickness of 10 μm to 50 μm, has a 100% modulus in at least one direction of preferably 1 to 5 N/20 mm-width.

The adhesive skin patch of the present invention has a pressure-sensitive adhesive layer on one side of the film base material for an adhesive skin patch. Specifically, it has a pressure-sensitive adhesive layer on one side of the film base material for an adhesive skin patch made of the above-mentioned ether-based urethane resin. It is preferable that the pressure-sensitive adhesive layer is formed from at least one kind selected from the group consisting of an acrylic pressure-sensitive adhesive consisting mainly of an acrylic acid ester, a silicone pressure-sensitive adhesive consisting mainly of polyorganosiloxane, and a urethane pressure-sensitive adhesive consisting mainly of polyether polyurethane and/or polyester polyurethane.

When the pressure-sensitive adhesive layer is to be formed form an acrylic pressure-sensitive adhesive, for example, an acrylic acid ester-based polymer is mixed with a carboxylic acid ester that is compatible with the acrylic acid ester-based polymer and a crosslinking agent and the resulting mixture is subjected to crosslinking treatment to obtain the objective pressure-sensitive adhesive layer. Note that the carboxylic acid ester has 16 or more carbon atoms and is liquid or paste at room temperature.

The acrylic acid ester-based polymer means a polymer that consists mainly of (meth)acrylic ester and is copolymerized with a monomer copolymerizable therewith as necessary. Preferable examples of the (meth)acrylic acid ester include (meth)acrylic acid alkyl esters in which the alkyl group has 2 or more carbon atoms and which has 2 or more and 18 or less carbon atoms. Specific examples thereof include ethyl, propyl, butyl, pentyl, hexyl, octyl, nonyl, decyl, dodecyl, etc. esters of (meth) acrylic acid. It is preferable that one or more from among these (meth) acrylic acid esters be used. The alkyl ester chains may be either linear or branched.

The monomers that are copolymerizable with the (meth)acrylic acid ester include, for example, carboxyl group-containing monomers such as (meth) acrylic acid, itaconic acid, and maleic acid, hydroxyl group-containing monomers such as 2-hydroxylethyl (meth)acrylate and 2-hydroxypropyl (meth)acrylate, alkoxy group-containing monomers such as methoxyethyl (meth) acrylate, ethoxyethyl (meth) acrylate, butoxyethyl (meth)acrylate, methoxypolyethylene glycol (meth) acrylate, and ethoxydiethylene glycol (meth) acrylate, styrene, styrene derivatives, vinyl monomers such as vinyl acetate and N-vinyl-2-pyrrolidone, and so on. One or more of these monomers may be used to copolymerize the (meth) acrylic acid esters therewith.

The acrylic acid ester-based polymer desirably has a glass transition temperature of 260K or less. By setting the glass transition temperature of the acrylic acid ester-based polymer at 260K or less, adhesion to the skin can be sufficiently exhibited so that the resultant pressure-sensitive adhesive layer is desirable as one for a pressure-sensitive adhesive sheet for medical use or hygine materials.

The acrylic acid ester-based polymer can be obtained by a conventional polymerization method such as a solution polymerization method, an emulsion polymerization method, a suspension polymerization method and so on. Also, the acrylic acid ester-based polymer can be obtained by radical polymerization by using a radical polymerization initiator such as a peroxide compound or an azo compound.

The carboxylic acid ester that is compatible with the acrylic acid ester-based polymer is preferably liquid or paste at room temperature. A pressure-sensitive adhesive layer formed by mixing a solid, e.g., waxy carboxylic acid ester may in some cases have a decreased adhesion.

In the present invention, gel-like pressure-sensitive adhesive layer can be obtained by mixing an acrylic acid ester-basedpolymer, acarboxylicacidester, andacrosslinking agent and forming crosslinked moiety in at least a portion thereof. The pressure-sensitive adhesive layer thus obtained can have a decreased elastic modulus in minute deformed regions, so that adhesion (wetting) of the surface of the pressure-sensitive adhesive layer to the unevenness of the surface of skin increases and sufficient adhesion to the surface of skin can be exhibited. In addition, when the adhesive skin patch is peeled from the skin, stress applied to the surface of skin can be released or dispersed. As a result, advantageous effects can be obtained in that substantially no physical stimulations are given onto the surface of skin when the adhesive skin patch is peeled off, while causing substantially no peeling off of the stratum corneum of the surface of skin, or minimized damages to the skin.

The carboxylic acid esters that can be preferably used in the present invention include esters of various fatty acids such as phthalic acid, maleic acid, adipic acid and stearic acid with alkyl alcohols, esters with polyhydric alcohols such as ethylene glycol and glycerol, and so on. For example, esters obtained by using monohydric alcohols such as ethyl myristate, isopropyl myristate, isopropyl palmitate, butyl stearate, isopropyl isostearate, hexyl laurate, cetyl lactate, myristyl lactate, diethyl phthalate, dioctyl phthalate, octyl dodecyl myristate, octyl dodecyl oleate, hexyl decyl dimethyloctanoate, cetyl 2-ethylhexanoate, isocetyl 2-ethylhexanoate, stearyl 2-ethylhexanoate, and dioctyl succinate, and esters obtained by using polyhydric alcohols, that is, dihydric or more alcohols such as propylene glycol dicaprylate, propylene glycol dicaprate, propylene glycol diisostearate, glyceryl monocaprylate, glyceryl tricaprylate, glyceryl tri-2-ethylhexannoate, glyceryl tricaprinate, glyceryl trilaurate, glyceryl triisostearate, glyceryl trioleate, and trimethylolpropane tri-2-ehtylhexanoate.

Note that the carboxylic acid esters used herein must have 16 or more carbon atoms. If the carboxylic acid esters have 15 or less carbon atoms, the film base material absorbs liquid components in large amounts so that swelling deformation of the film base material occurs.

In the present invention, when the above-mentioned carboxylic acid esters are blended, at least one kind from among them is dissolved in the acrylic acid ester-based polymer. A blending amount of the carboxylic acid is not particularly limited. For example, it is preferable that the carboxylic acid ester in the range of 30 to 100 mass parts be added to 100 mass parts of the acrylic acid ester-based polymer.

In the present invention, when the acrylic acidester-based polymer having dissolved therein the above-mentioned carboxylic acid ester is used, it is necessary that crosslinked moiety be formed in at least a portion of the polymer. To form crosslinked moiety, crosslinking treatment is performed. For example, chemical crosslinking treatment may be performed by using an organic peroxide compound, an isocyanate compound, an organic metal salt, a metal chelate, an epoxy compound or the like or physical crosslinking treatment may be performed by using ionizing radiation.

The resin composition (pressure-sensitive adhesive) that forms the pressure-sensitive adhesive layer may be blended with various additives, e.g., plasticizers such as glycerol and polyethylene glycol, water-soluble or water-absorbing resins such as polyacrylic acid and polyvinylpyrrolidone, tackifiers such as rosin-based, terpene-based, petroleum-based tackifiers, various types of softening agents, and various additives such as fillers, pigments. In particular, when carboxylic acid esters having unsaturated bonds are used as the carboxylic acid ester, it is feared that the physical properties will change due to oxidation deterioration caused by oxygen in the atmosphere, thus failing to exhibit desired characteristics, so that it is preferable that conventional antioxidants are blended in the resin composition (pressure-sensitive adhesive).

It is preferable that the thickness of the pressure-sensitive adhesive layer is set in the range of 10 μm to 100 μm. If the thickness of the pressure-sensitive adhesive layer is less than 10 μm, itmayhappen thatno sufficient adhesion is exhibited during application to the skin. On the other hand, if the thickness of the pressure-sensitive adhesive layer is above 100 μm, it may happen that permeability of water vapor on levels that are required for adhesive skin patches cannot be obtained.

When the adhesive skin patch is applied to a surface of skin of human body, the adhesive skin patch must have a moisture permeability of 600 g/$m^2$·24h·40°C·30%RH or more when stored at 40°C and 30% RH for 24 hours although some variations may occur depending on difference among individuals or sites where the adhesive skin patch is applied. If the pressure-sensitive adhesive sheet (adhesive skin patch) having moisture permeability less than 600 g/$m^2$·24h·40°C·30%RH is applied to the skin for 1 week or more, continuous non-breathing occurs, thus causing skin irritation. It is preferable that the moisture permeability of the adhesive skin patch is set in the range of 800 g/$m^2$·24h·40°C·30%RH to 2,400 g/$m^2$·24h·40°C·30%RH.

It is preferable that the filmbase material for an adhesive skin patch of the present invention has moisture permeability after the film base material is provided thereon with a pressure-sensitive adhesive layer, i.e., as moisture permeability of adhesive skin patch of 1,000 g/$m^2$·24h·40°C·30%RH or more by the dry method and 2,000 g/$m^2$·24h·40°C·30%RH or more by the wet method.

Further, it is desirable that the adhesive skin patch has excellent water resistance. Swelling, wrinkles, or lifting off from the adherend, if any, when the adhesive skin patch is immersed in water are not preferable since it is feared that the sealability given by the adhesive skin patch will be deteriorated.

Preferably, the film base material for an adhesive skin patch of the present invention has less deformation after a pressure-sensitive adhesive layer is formed thereon. If the deformation is too large, it may happen that pressure-sensitive adhesive tapes cannotbe realized or adhesive skinpatches cannot be formed. Therefore, the film base material for an adhesive skin patch preferably has absorbability of a liquid component of the pressure-sensitive adhesive that forms the pressure-sensitive adhesive layer, for example, glyceryl tricaprylate (hereinafter, sometimes abbreviated as "GTC") of 50% or less and a deformation ratio of 10% or less.

In the present invention, the adhesive skin patch can be used for forming medical tapes or sheets such as adhesive bandages. For example, the adhesive skin patch may be cut to an appropriate size to form adhesive bandages, covering materials for covering wounded portions, protectors used after surgical operations, medical tapes or sheets such as covers, e.g., pads for insertion needles of catherter, gauze, or the adhesive skin patch may be combined with other base materials to form medical products such as tapes for fixing and tapes for holding instruments. Note that the base material for an adhesive skin patch and the adhesive skin patch can be used for uses other than medical uses so far as they relate to uses involving application to the skin. For example, the base material for an adhesive skin patch and the adhesive skin patch can be used for pressure-sensitive pierces, tapes for tattoo, tapes for fixing wigs, tapes for artificial hair grafts and so on.

EXAMPLES

Hereinafter, the present invention will be described in detail by way of examples. However, the present invention should not be considered to be limited thereto and various applications may be possible within the scope not departing the technical concept of the present invention. In the following examples, all parts are by weight. In addition, the measuring methods and evaluation methods used in the following examples are indicated below.

<Measuring methods and evaluation methods>

(1) Moisture permeability of film base material (Dry method)

20 ml of purified water was charged in a glass-made vessel (weighing bottle) having an inner diameter of 40 mm and a height of 40 mm, and then a film base material for an adhesive skin patch cut into a disk of 50 mm in diameter was applied and fixed to the opening of the vessel. After measuring total weight (W1) of the vessel to which the film base material was applied, this was placed in a homeostat at 40°C and a relative humidity of 30%RH and measured for a total weight (W2) of the vessel after standing for 24 hours. The moisture permeability of the filmbasematerial was calculatedbasedon the following equation. Note that an assumption was made that the moisture permeability was inversely proportional to the thickness and results were converted to values of those having a thickness of 30 μm.

$$\text{Moisture Permeability (g/m}^2\text{·24h·40°C·30\%RH)} = (W1 - W2)/(0.02 \times 0.02 \times \pi)$$

(2) Moisture permeability of adhesive skin patch (Dry method)

In the same manner as in the measurement of the moisture permeability of the film base material in (1) above, moisture permeability was obtained except that the adhesive skin patch was fixed such that a pressure-sensitive adhesive layer was arranged on the side of water (water vapor), that is, in contact with the opening of the glass-made vessel.

(3) Moisture permeability of film base material (filter paper cover method, Wet method)

Disks of 60 mm in diameter were punched from a film base material. Six pieces of circular filter paper of 30 mm in diameter were overlapped and mounted on a 70 mm-square aluminum plate and the six pieces of circular filter paper were impregnated with 1 mL of purified water. Then, the punched film base material of 60 mm in diameter was laminated so that it covered the filter paper and fixed to the aluminum plate. The thus obtained aluminum plate having fixed thereon the film base material was stored in a homeostat at 40°C and a relative humidity of 30%RH for 1 hour or more. The weights of the aluminum plate with the film base material before and after the storage were measured (W3: weight before storage, and W4: weight after storage), and the moisture permeability of the film base material was calculated based on the following equation. Note that an assumption was made that the moisture permeability was inversely proportional to the thickness and results were converted to values of those having a thickness of 50 μm.

$$\text{Moisture Permeability (g/m}^2\cdot\text{24h}\cdot\text{40}°\text{C}\cdot\text{30\%RH)} = (W3 - W4)/(0.015 \times 0.015 \times \pi)$$

(4) Moisture permeability of adhesive skin patch (filter paper cover method, Wet method)

In the same manner as in the measurement of the moisture permeability of the film base material in (3) above, moisture permeability of adhesive skin patch was obtained.

(5) Absorbability of liquid components (deformation ratio, absorbability)

Immediately after preparation of a film base material for an adhesive skin patch, the film base material was cut to pieces of a size of 50 mm × 50 mm to make a sample. The sample was measured for a length of one side and weight (here, indicated as "initial length" and "initial weight"). After the sample was immersed in glyceryl tricaprylate for 3 days, taken out and wiped off of the liquid that attached to the surface thereof, length of one side and weight of the sample were measured (here, indicated as "length after immersion" and "weight after immersion"). Based on the following equations, deformation ratio and absorbability of the film base material for an adhesive skin patch were calculated.

$$\text{Deformation ratio (\%)} = [(\text{Length after immersion - Initial length})/\text{Initial length}] \times 100$$

$$\text{Absorbability (\%)} = [(\text{Weight after immersion - Initial weight})/\text{Initial weight}] \times 100$$

(6) Water swellability

A film base material for an adhesive skin patch was cut to a size of 50 mm × 50 mm and a gauge line of 50 mm in length was marked thereon in the diagonal direction. This was immersed in purified water at 40°C for 5 minutes and then taken out. Immediately thereafter, the length of the gauge line (L) was measured. Water swellability (%) was obtained based on the following equation.

$$\text{Water Swellability (\%)} = \{(L - 50)/50\} \times 100$$

(7) Water resistance of adhesive skin patch

An adhesive skin patch was cut to a size of 40 mm × 40 mm and four corners thereof were cut off so as to form round corners. The thus-cut adhesive skin patch was applied on an aluminum plate such that no air bubbles were entrained. Then, the aluminum plate on which the adhesive skin patch was applied was immersed in purified water at 40°C for 5 minutes and then taken out. The adhesive skin patch after the immersion was observed with naked eye and evaluated with ranks : "×" indicating that wrinkles or lifting up occurred; "Δ" indicating that it is still usable in spite of occurrence of a little wrinkles or lifting up; and "○" indicating that it retained acceptable application state without occurrence of wrinkles or lifting up.

(8) Tensile strength

A sample of 20 mm in width × about 100 mm in length was taken and a gauge line was marked at a point corresponding to a length of 50 mm (a distance between chucks being 50 mm). This sample was drawn at a drawing speed of 200 mm/minute and the maximum stress at break was defined as tensile strength (N/20 mm-width).

(9) Elongation

A sample of 20 mm in width × about 100 mm in length was taken and a gauge line was marked at a point corresponding to a length of 50 mm (a distance between chucks being 50 mm). This sample was drawn at a drawing speed of 200 mm/minute and the length of elongation at break was obtained based on the following equation.

$$\text{Elongation (\%)} = \{\text{Length of gauge line at break (mm)} - 50 \text{ mm}\} / 50 \text{ mm}\} \times 100$$

(10) Tear strength

Tear strength was measured based on the B method described in JIS-K-6301-1995. That is a sample type B was drawn at a drawing speed of 500 mm/minute until it was broken and the maximum force required for tearing the sample then was measured. Tear strength of the sample was obtained based on the following equation.

$$\text{Tear Strength (N/cm)} = (\text{Maximum tear force (N)})/(\text{Thickness of sample (cm)})$$

(11) 100% Modulus

A sample of 20 mm in width × about 100 mm in length was taken and a gauge line was marked at a point corresponding to a length of 50 mm (a distance between chucks being 50 mm). This sample was drawn at a drawing speed of 200 mm/minute and the tensile stress (N/20 mm-width) when the sample was drawn 100% was measured.

(Example I-1)

In a reactor equipped with a condenser, a heater, a thermometer and a stirrer were charged and mixed 38 g of polyoxytetramethylene glycol (OTMG) having a weight-average molecular weight of 1,000, 26 g of polyethylene glycol (PEG) having a weight-average molecular weight of 2,000, and 6 g of 1,4-butanediol (BD) as polyols. While stirring the resultant mixture so that the temperature was 70°C, 30 g of methylene diphenyl diisocyanate (MDI) at 50°C was added as polyisocyanate and the resultant was stirred for 5 minutes. Thereafter, the reaction product was transferred to a tray, which was placed in a hot-air drying chamber and aged at 140°C for 5 hours to obtain a bulky ether-based urethane resin. The obtained bulky ether-based urethane resin was pulverized and dissolved in N,N-dimethylformamide (DMF) to prepare a solution having a concentration of 30%. The solution was cast on a release-treated surface of a release-treated polyester film (38 μm in thickness) to a dry thickness of 30 μm by using an applicator, dried at 160°C for 5 minutes in a hot-air drying chamber to obtain a film base material for an adhesive skin patch made of the ether-based urethane resin.
Then, a pressure-sensitive adhesive layer was formed on the obtained film base material for an adhesive skin patch.
Isononyl acrylate (NA), methoxyethyl acrylate (MEA), and acrylic acid (AA) were copolymerized to obtain an acrylic acid alkyl ester-based polymer. In this case, however, the blending ratios to obtain the acrylic acid alkyl ester-based polymer were NA:MEA:AA = 65:30:5 in weight ratios. 100 parts by weight (solids content) of the obtained acrylic acid alkyl ester-based polymer, 60 parts by weight of glyceryl tricaprylate (GTC) as carboxylic acid ester component, and 0.2 part by weight of trifunctional isocyanate compound as crosslinking agent component were mixed in toluene to prepare a solution for a pressure-sensitive adhesive layer (concentration: 33%). The solution for a pressure-sensitive adhesive layer was coated on a release-treated surface of a release-treated paper separator to a dry thickness of 30 μm and dried at 110°C for 3 minutes in a hot-air drying chamber to form a pressure-sensitive adhesive layer.

A film base material made of the prepared ether-based urethane resin was applied onto the obtainedpressure-sensitive adhesive layer and then stored in an atmosphere at 60°C for 3 days in a hot-air drying chamber to perform aging to complete crosslinking reaction of the pressure-sensitive adhesive layer, thereby preparing an adhesive skinpatch for a dressingmaterial .

The obtained film base material for an adhesive skin patch was measured for moisture permeability (Dry method). Also, the film base material for an adhesive skin patch was measured for absorbability of liquid component (GTC). The results obtained are shown in Table 1.

(Example I-2)

A film base material for an adhesive skin patch was in the same manner as in that in Example I-1 except that in Example I-1, 6 g of polyoxytetramethylene glycol (OTMG) having a weight-average molecular weight of 1,000, 40 g of polyethylene glycol (PEG) having a weight-average molecular weight of 2 , 000 , 10 g of polypropylene glycol (PPG) having a weight-average molecular weight of 2,000, and 8 g of 1,4-butanediol (BD) as polyols were charged and mixed, and while stirring the resultant mixture so that the temperature was 70°C, 36 g of methylene diphenyl diisocyanate (MDI) at 50°C was added, followed by stirring the resultant for 5 minutes.

Further, an adhesive skin patch was prepared in the same manner as that in Example I-1 using the obtained film base material for an adhesive skin patch.

The obtained film base material for an adhesive skin patch and the adhesive skin patch were measured and evaluated in the same manner as that in Example I-1. The results are shown in Table 1.

(Example 1-3)

A film base material for an adhesive skin patch was prepared in the same manner as in that in Example I-1 except that in Example I-1, 58 g of polyoxytetramethylene glycol (OTMG) having a weight-average molecular weight of 1,000 and 10 g of 1,4-butanediol (BD) as a polyol were charged and mixed, and while stirring the resultant mixture so that the temperature was 70°C, 32 g of methylene diphenyl diisocyanate (MDI) at 50°C was added, followed by stirring the resultant for 5 minutes.

Further, an adhesive skin patch was prepared in the same manner as that in Example I-1 using the obtained film base material for an adhesive skin patch.

The obtained film base material for an adhesive skin patch and the adhesive skin patch were measured and evaluated in the same manner as that in Example I-1. The results are shown in Table 1.

(Comparative Example I-1)

A 25-μm-thick polyether polyamide film was provided as a pore-less moisture permeable film. A pressure-sensitive adhesive layer was formed on one surface of the film and then an adhesive skin patch was prepared therefrom in the same manner as in that in Example I-1.

The polyether polyamide film and the prepared adhesive skin patch were measured and evaluated in the same manner as that in Example I-1. The results are shown in Table 1.

Table 1

| | Isocyanate component | Diol component | Moisture Permeability (Dry method) (g/m$^2$·24hrs· 40°C-30%RH) | Liquid component absorbability (%) | |
|---|---|---|---|---|---|
| | | | | Deformation ratio | Absorbability |
| Example I-1 | MDI | OTMG PEG BD | 1,900 | 3 | 12 |
| Example I-2 | MDI | OTMG PEG PPG BD | 2,200 | 2 | 14 |
| Example I-3 | MDI | OTMG BD | 1,000 | 6 | 25 |
| Compara tive Example I-1 | Polyether polyamide-based resin | | 2,100 | 15 | 60 |

Table 1 clearly indicates that the film base materials for an adhesive skin patch of Examples I-1 to I-3 had moisture permeability of 1,000 g/m$^2$·24h·40°C·30%RH or more, thus exhibiting excellent moisture permeability. In particular, the film base materials for an adhesive skin patch of Examples I-1 and I-2 in which PEG was contained as the diol component had moisture permeability of 1,900 g/m$^2$·24h·40°C·30%RH or more, thus exhibiting particulary excellent moisture permeability.

On the other hand, the film base material for an adhesive skin patch made of polyether amide of Comparative Example I-1 had excellent moisture permeability but showed very high absorbability of glyceryl tricaprylate as high as 60%, thus showing a considerable deformation due to absorption of liquid components, so that no acceptable adhesive skin patch could be realized.

Further, measurement of the adhesive skin patches of Examples I-1 and I-2 for moisture permeability indicated that the moisture permeability of the adhesive skin patch of Example I-1 was 1, 030 g/m$^2$·24h·40°C·30%RH and the moisture permeability of the adhesive skin patch of Example I-2 was 1,180 g/m$^2$·24h·40°C·30%RH. That is, the base film materials for an adhesive skin patch of Examples I-1 and I-2 had excellent moisture permeability even when they had a pressure-sensitive adhesive layer thereon.

Note that the adhesive skin patches of Examples I-1 to I-3 had suitable adhesion and excellent followability to the skin and the like.

(Example II-1)

The film base material for an adhesive skin patch and the adhesive skin patch prepared in Example I-1 were measured for moisture permeability, respectively. Also, water swellability of the film base material for an adhesive skin patch and water resistance of the adhesive skin patch were evaluated. The results obtained are shown in Table 2.

(Example II-2)

The film base material for an adhesive skin patch and the adhesive skin patch prepared in Example I-3 were measured for moisture permeability, respectively. Also, water swellability of the film base material for an adhesive skin patch and water resistance of the adhesive skin patch were evaluated. The results obtained are shown in Table 2.

(Example II-3)

A filmbase material for an adhesive skin patch was prepared in the same manner as in that in Example I-1 except that in Example I-1, 22 g of polyoxytetramethylene glycol (OTMG) having a weight-average molecular weight of 1,000, 33 g of polyethylene glycol (PEG), 5 g of polypropylene glycol (PPG), and 7 g of 1,4-butanediol (BD) as polyols were charged and mixed, and while stirring the resultant mixture so that the temperature was 70°C, 33 g of methylene diphenyl diisocyanate (MDI) at 50°C was added as polyisocyanate, followed by stirring the resultant for 5 minutes.

Further, an adhesive skin patch was prepared in the same manner as that in Example I-1 using the obtained film base material for an adhesive skin patch.

The obtained film base material for an adhesive skin patch and the adhesive skin patch were measured for moisture permeability, respectively. Also, water swellability of the film base material for an adhesive skin patch and water resistance of the adhesive skin patch were evaluated. The results obtained are shown in Table 2.

(Example II-4)

The film base material for an adhesive skin patch and the adhesive skin patch prepared in Example I-2 were measured for moisture permeability, respectively. Also, water swellability of the film base material for an adhesive skin patch and the water resistance of adhesive skin patch were evaluated. The results obtained are shown in Table 2.

Table 2

| | Water swellability (%) | Moisture permeability (Wet method) (g/m²·24h·40°C·30%RH) | Moisture permeability (Dry method) (g/m²·24h·40°C·30%RH) | Moisture permeability of adhesive skin patch (g/m²·24h·40°C·30%RH) (Dry, Wet) | | Water Resistance |
|---|---|---|---|---|---|---|
| ExamPle II-1 | 0 | 4,400 | 1,900 | 1,030 | 1,390 | ○ |
| Example II-2 | 0 | 3,900 | 1,000 | 600 | 1,050 | ○ |
| Example II-3 | 0 | 7,100 | 1,800 | 1,120 | 2,660 | Δ |
| Example II-4 | 10 | 15,000 | 2,200 | 1,180 | 2,780 | × |

Table 2 clearly indicates that the film base materials for an adhesive skin patch of Examples II-1 to II-4 had moisture permeability (Dry method) of 1,000 g/m²·24h·40°C·30%RH or more, thus exhibiting excellent moisture permeability. Tables 2 also indicates that the film base materials for an adhesive skin patch of Examples II-1 to II-4 had excellent moisture permeability by the Wet method and further that even when the adhesive skin patches on which a pressure-sensitive adhesive layer was formed had excellent moisture permeability. The adhesive skin patches of Examples II-1 and II-2 had excellent water resistance while the adhesive skin patch of Example II-3 was above practical usable level. In particular, the film base material for an adhesive skin patch of Examples II-1 hadmoisture permeability (Dry method) of 1,900 g/m²·24h·40°C·30%RH or more, thus having particularly excellent moisture permeability, and the adhesive skinpatches onwhich apressure-sensitive adhesive layerwas formed had excellent moisture permeability (Dry method, Wet method) and excellent water resistance, thus proving to be a film base material with well-balanced properties.

Note that the film base material for an adhesive skin patch of Examples II-4 having water swellability of above 5% showed considerable deformation due to absorption of moisture, thus having poor water resistance but it had excellent moisture permeability and as will be apparent from the results of Example I-2 in Table 1, it had excellent absorbability of liquid components, so that it can be used for limited applications.

In addition, the adhesive skin patches of Examples II-1 to II-4 had suitable adhesion and excellent followability to the skin and the like.

(Example III-1)

The following measurements were made on the film base material for an adhesive skin patch prepared in Example I-1. That is, tensile strength, elongation, tear strength and 100% modulus in the direction of flow of the film base material for an adhesive skin patch (MD: machinery direction) and in the direction perpendicular thereto (TD: transverse direction), respectively, were measured. The results are shown in Table 3.

Table 3

| | Thickness (µm) | Tensile strength (N/20 mm) MD TD | | Elongation (%) MD TD | | Tear strength (N/cm) MD TD | | 100% Modulus (N/20 mm) MD TD | |
|---|---|---|---|---|---|---|---|---|---|
| Example III-1 | 31 | 20.3 | 19.5 | 770 | 780 | 660 | 630 | 2.2 | 2.1 |

Table 3 clearly indicates that the film base material for an adhesive skin patch of Example III-1, that is, Example I-1, had tensile strengths in the TD and in the MD within the range of 5 to 30 N/20 mm-width, elongations in the TD and in the MD direction of within the ranges of 400% to 1,000%, and tear strengths in the TD and in the MD within the range of 400 to 1,000 N/10 mm-width.

According to the present invention, film base materials for an adhesive skin patch having flexibility sufficient to enable them to follow up the skin and the like and excellent moisture permeability can be provided.

The adhesive skin patches of the present invention can be used in the form of sheets, tapes having various dimensions and can be stored in the form of rolls. The adhesive skin patches can be used in various fields of skin patches, for example, in medical and hygienic fields, external uses and the like. Specifically, the adhesive skin patches of the present invention can be advantageously used for adhesive bandages, pressure-sensitive dressings, dressing materials, and the like.

**Claims**

1.   A film base material for an adhesive skin patch, comprising an ether-based urethane resin obtained from at least one member selected from the group consisting of polyoxytetramethylene glycol, butanediol, polyethylene glycol, and polypropylene glycol as a diol component, and methylene diphenyl-diisocyanate as an isocyanate component.

2.   The film base material for an adhesive skin patch according to claim 1, wherein the diol component comprises polyoxytetramethylene glycol, and polyethylene glycol and/or polypropylene glycol.

3.   The film base material for an adhesive skin patch according to claim 2, wherein the ether-based urethane resin contains 5 to 60% by weight of the polyoxytetramethylene glycol and 10 to 50% by weight of the polyethylene glycol.

4.   The film base material for an adhesive skin patch according to any one of claims 1 to 3, wherein the film base material comprises a film consisting of a urethane based resin and has a water swellability of 5% or less expressed as a change in length when immersed in water at 40°C for 5 minutes.

5.   The film base material for an adhesive skin patch according to claim 4, wherein the water swellability of the film is substantially 0%.

6.   The film base material for an adhesive skin patch according to any one of claims 1 to 5, wherein the film base material has a thickness of 10 µm to 50 µm.

7.   The film base material for an adhesive skin patch according to any one of claims 1 to 6, wherein the film base material comprises an ether-based urethane resin film having a thickness of 10 µm to 50 µm, and has a tensile strength in at least one direction of 5 to 30 N/20 mm-width, an elongation in at least one direction of 400% to 1,000%, and a tear strength in at least one direction of 400 to 1,000 N/cm-thickness.

8.   The film base material for an adhesive skin patch according to claim 7, wherein the film base material has a 100% modulus in at least one direction of 1 to 5 N/20 mm-width.

9.   The film base material for an adhesive skin patch according to any one of claims 1 to 8, wherein the film base material has a moisture permeability of 800 to 4,000 g/m$^2$·24hrs.

10.   An adhesive skin patch comprising the film base material for an adhesive skin patch according to any one of claims 1 to 9, and a pressure-sensitive adhesive layer on one side of the film base material.

11.   The adhesive skin patch according to claim 10, wherein the pressure-sensitive adhesive layer comprises at least one pressure-sensitive adhesive selected from the group consisting of an acrylic pressure-sensitive adhesive consisting mainly of a (meth)acrylic acid ester, a silicone pressure-sensitive adhesive consisting mainly of polyorganosiloxane, and a urethane pressure-sensitive adhesive consisting mainly of polyether polyurethane and/or polyester polyurethane.

**European Patent Office**

## EUROPEAN SEARCH REPORT

Application Number

EP 05 00 2021

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 367 109 A (NITTO DENKO CORPORATION) 3 December 2003 (2003-12-03) * paragraph [0041]; claims 8-10; examples * ----- | 1-11 | A61L15/26 |
| X | WO 85/05373 A (THORATEC LABORATORIES CORPORATION) 5 December 1985 (1985-12-05) | 1-9 | |
| Y | * page 8; claim 11; examples 4-7 * ----- | 1-11 | |
| X | PATENT ABSTRACTS OF JAPAN vol. 1998, no. 03, 27 February 1998 (1998-02-27) & JP 09 299473 A (NIPPON ZEON CO LTD), 25 November 1997 (1997-11-25) | 1-5,8,9 | |
| Y | * abstract * ----- | 1-11 | |
| X | EP 0 633 277 A (TAKEDA BADISCHE URITHANE INDUSTRIES LTD; TAKEDA BADISCHE URETHANE INDU) 11 January 1995 (1995-01-11) * page 2, line 30 - line 36; examples; table 1 * ----- | 1-9 | |
| X | PATENT ABSTRACTS OF JAPAN vol. 016, no. 490 (C-0994), 12 October 1992 (1992-10-12) & JP 04 180913 A (TOYO TIRE & RUBBER CO LTD), 29 June 1992 (1992-06-29) * abstract * -& DATABASE WPI Section Ch, Week 199232 Derwent Publications Ltd., London, GB; Class A25, AN 1992-264409 XP002330644 & JP 04 180913 A (TOYO RUBBER IND CO LTD) 29 June 1992 (1992-06-29) * abstract * ----- -/-- | 1-9 | **TECHNICAL FIELDS SEARCHED (Int.Cl.7)** A61L |

The present search report has been drawn up for all claims

| Place of search | Date of completion of the search | Examiner |
|---|---|---|
| Munich | 6 June 2005 | Winger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 05 00 2021

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 015, no. 474 (C-0890), 3 December 1991 (1991-12-03) & JP 03 203920 A (DAINICHISEIKA COLOR & CHEM MFG CO LTD; others: 01), 5 September 1991 (1991-09-05) * abstract * ----- | 1-9 | |
| | | | TECHNICAL FIELDS SEARCHED (Int.Cl.7) |

The present search report has been drawn up for all claims

| Place of search | Date of completion of the search | Examiner |
|---|---|---|
| Munich | 6 June 2005 | Winger, R |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 00 2021

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2005

| Patent document cited in search report | | Publication date | Patent family member(s) | | Publication date |
|---|---|---|---|---|---|
| EP 1367109 | A | 03-12-2003 | JP | 2003342541 A | 03-12-2003 |
| | | | AT | 295398 T | 15-05-2005 |
| | | | AU | 2003204326 A1 | 18-12-2003 |
| | | | CA | 2429388 A1 | 29-11-2003 |
| | | | EP | 1367109 A1 | 03-12-2003 |
| | | | US | 2003224160 A1 | 04-12-2003 |
| WO 8505373 | A | 05-12-1985 | AT | 67226 T | 15-09-1991 |
| | | | AU | 4357385 A | 13-12-1985 |
| | | | CA | 1257425 A1 | 11-07-1989 |
| | | | DE | 3584077 D1 | 17-10-1991 |
| | | | EP | 0183790 A1 | 11-06-1986 |
| | | | JP | 5004407 B | 20-01-1993 |
| | | | JP | 61502260 T | 09-10-1986 |
| | | | WO | 8505373 A1 | 05-12-1985 |
| JP 09299473 | A | 25-11-1997 | NONE | | |
| EP 0633277 | A | 11-01-1995 | JP | 3294913 B2 | 24-06-2002 |
| | | | JP | 7018052 A | 20-01-1995 |
| | | | AT | 157675 T | 15-09-1997 |
| | | | DE | 69405325 D1 | 09-10-1997 |
| | | | DE | 69405325 T2 | 08-01-1998 |
| | | | DK | 633277 T3 | 20-10-1997 |
| | | | EP | 0633277 A1 | 11-01-1995 |
| | | | ES | 2106418 T3 | 01-11-1997 |
| | | | GR | 3025371 T3 | 27-02-1998 |
| JP 04180913 | A | 29-06-1992 | JP | 2665626 B2 | 22-10-1997 |
| JP 4180913 | A | 29-06-1992 | JP | 2665626 B2 | 22-10-1997 |
| JP 03203920 | A | 05-09-1991 | JP | 2967540 B2 | 25-10-1999 |

EPO FORM P0459

For more details about this annex : see Official Journal of the European Patent Office, No. 12/82